# EUROPEAN PATENT SPECIFICATION

(11) **EP 0 231 091 B1**
(45) Date of publication and mention of the grant of the patent: **31.03.1993**
(21) Application number: 87300454.3
(22) Date of filing: 20.01.1987
(51) Int. Cl.: A61K 9/10, A61K 31/02

(54) **Stable emulsions of highly fluorinated organic compound**
Stabile Emulsionen von stark fluorierten, organischen Verbindungen
Emulsions stables de composés organiques hautement fluorés

(30) Priority: 24.01.1986 US 822291; 09.07.1986 US 883713
(43) Date of publication of application: 05.08.1987
(73) Proprietor: CHILDREN'S HOSPITAL MEDICAL CENTER, Cincinnati Ohio 45229-2899 (US); HemaGen PFC, San Francisco California 94108 (US)
(72) Inventor: Clark, Leland C., Jr., Cincinnati, Ohio 45229 (US); Shaw, Robert F., San Francisco California 94133 (US)
(74) Representative: Allen, Oliver John Richard

(56) References cited:
- EP-A- 0 051 526
- EP-A- 0 080 716
- EP-A- 0 089 232
- EP-A- 0 158 996
- EP-A- 0 220 153
- WO-A-84/03624
- FR-A- 2 494 992
- US-A- 3 962 439
- US-A- 4 252 827

## Description

This invention relates to stable, physiologically acceptable emulsions of highly fluorinated organic compounds and to processes of making and using them. Such emulsions are especially useful in compositions for use as oxygen transport agents, "artificial bloods" or red blood cell substitutes and as contrast agents for biological imaging.

Highly fluorinated organic compounds are well known to be chemically and pharmaceutically inert and to be capable of dissolving and transporting large amounts of oxygen. These properties make them potentially useful as oxygen transport agents, "artificial bloods" or red blood cell substitutes and as contrast agents for various imaging modalities, such as nuclear magnetic resonance, ultrasound, and x-ray. For medical uses that require intravascular injection, highly fluorinated organic compounds must be dispersed as physiologically acceptable emulsions as they are too hydrophobic to be miscible with blood. (See, e.g., L.C. Clark, Jr. et al., "Emulsions Of Perfluorinated Solvents For Intravascular Gas Transport", Fed. Proc., 34(6), pp. 1468-77 (1975); K. Yokoyama et al., "A Perfluorochemical Emulsion As An Oxygen Carrier", Artif. Organs (Cleve), 8(1), pp. 34-40 (1984); and United States Patents 4,110,474 and 4,187,252).

To date, however, the medical usefulness of such emulsions as "artificial bloods" or blood substitutes, oxygen transport agents or contrast agents for biological imaging has not been as successful as hoped. This results from the fact that in practice it has not been previously possible to make emulsions that are both stable and incorporate the relatively large amounts of highly fluorinated organic compounds that are required in clinical practice where the total volume of emulsion that can be administered is limited, e.g., as "artificial bloods". Moreover, it has not been previously possible to make such emulsions using highly fluorinated organic compounds that are excreted from the body within a clinically acceptable time period (see United States Patent 3,911,138). Finally, even those, admittedly less than therapeutically acceptable, compositions that have been available to date are difficult to sterilize because of their instability at high temperature.

Various attempts have been made to prepare stable physiologically acceptable emulsions containing high concentrations of clinically suitable highly fluorinated organic compounds but none has been successful. To date only one fluorocarbon emulsion has reached clinical testing as an "artificial blood" and that is "Fluosol DA 20%", which is about a 12% by volume emulsion of two fluorocarbons --perfluorodecalin and perfluorotripropylamine -- in a mixture of two surfactants -- yolk phospholipid and Pluronic F-68. The disadvantages of Fluosol DA 20% are firstly that it is not stable in the liquid state and must be stored frozen (Yokoyama et al., supra). Secondly, the required presence of the perfluorotripropylamine in this emulsion, to help "stabilize" it, disadvantages the emulsion's medical usefulness because the half-life of the perfluorotripropylamine in the liver and other body tissues is longer than desirable (see, e.g., K. Yokoyama et al., supra). Finally, because this emulsion contains only about 12% fluorocarbon by volume, it is much less therapeutically effective than desired because of its low oxygen content capacity (see, e.g., "Fluosol-DA As A Red Cell Substitute In Acute Anemia", N.E. Jour. Med., 314, pp. 1653-66 (1986)).

Therefore, the medical and non-medical uses of highly fluorinated organic compounds as effective oxygen transport agents, "artificial bloods" or red blood cell substitutes, and contrast agents for biological imaging is still a long sought and important goal.

The physiologically acceptable emulsions in accordance with the invention comprise at least one highly fluorinated organic compound, an oil that is not substantially surface active and not significantly soluble in water, a surfactant and water.

The emulsions of the invention are stable at room temperature for long periods of time. They exhibit substantially no phase separation and substantially no change in particle or droplet size distribution during storage. Moreover, they permit the use of highly fluorinated organic compounds that exhibit acceptably rapid excretion times from the liver and other body tissues, and, they permit the use of high concentrations of fluorocarbons thereby producing the high oxygen content capacity emulsions required for use of the emulsions of this invention as therapeutically effective blood substitutes. Finally, because of their stability, the emulsions of this invention may be sterilized by heating them to high temperature, for example, 115^{o}C for 15 min. Even such harsh conditions do not cause phase separation of the emulsions of this invention. As a result of these novel and unexpected properties, the emulsions of this invention solve the long standing problems relating to prior fluorocarbon containing compositions.

This inventon also includes methods of making these emulsions and methods and compositions of using them as oxygen transport agents, "artificial bloods" or red blood cell subsitutes, and contrast agents for biological imaging.

This invention also includes the use of perfluoroindane or an emulsion thereof as a gas transport agent or NMR contrast agent in animals without causing gas or vapor pulmonary embolism.

We believe that the emulsions of this invention may have the highly fluorinated organic compound dispersed in oil and that oil-fluorocarbon combination emulsified in the water and surfactant. However, other possible phases and interfaces are also within the scope of this invention.

Among the highly fluorinated organic compounds that are useful in the emulsions and processes of this invention are those previously said to be useful as oxygen transport agents, "artificial bloods" or red blood cell substitutes, and contrast agents for biological imaging. These include, for example, perfluorocarbons, partially fluorinated hydrocarbons and derivatives and mixtures of them. For example, among the fluoro-containing compounds useful in the emulsions of this invention are 9-18C perfluorohydrocarbons, e.g., perfluorodecalin, perfluoro-trimethylbicyclo[3.3.1]nonane and perfluoro-2,2,4,4-tetramethylpentane, 9-12C perfluoroamines, e.g., perfluorotripropylamine, perfluorotributylamine, perfluorodimethyladamantane, perfluoro-1-aza-tricylic amines, bromo- or iodo-substituted fluorocarbons, and F-4-methyloctahydroquinolidizine. Such compounds are described, for example, in United States Patents 3,962,439; 3,493,581; 4,110,474; 4,186,253; 4,187,252; 4,252,827; 4,423,077; 4,443,480; 4,534,978 and 4,542,147, European Patent Applications 80710 and 158,996, British Patent Specification 1,549,038 and German Offen. 2,650,586. Mixtures of any of these highly fluorinated organic compounds may also be used in the emulsions and processes of this invention.

Preferably, the emulsions of this invention contain one or more perfluorocarbons and most preferably a fluorocarbon selected from the group consisting of perfluorodecalin, perfluorodimethyladamantane,perfluorooctylbromide, perfluoro-4-methyl-octahydroquinolidizine, perfluoro-N-methyl-decahydroquinoline, F-methyl-1-oxa-decalin, perfluoro-bicyclo [5.3.0]decane, perfluoro-octahydroquinolidizine, perfluoro-5,6-dihydro-5-decene, perfluoroindane, perfluorotrimethylcyclohexane, perfluoroisopropylcyclohexane and perfluoro-4,5-dihydro-4-octene. For use as a contrast agent for biological imaging perfluorooctylbromide is one of the preferred highly fluorinated organic compounds according to this invention.

The highly fluorinated organic compounds or mixture of such compounds may comprise up to about 75% (by volume) of the emulsions of this invention. Preferably, the emulsions of this invention comprise from 10% to about 70% (by volume) of the fluorocarbon. When the emulsions are to be used as "artificial bloods" or red blood cell substitutes, the fluoro-containing compounds are preferably present in as high a volume concentration as possible. However, 40% (by volume) is often preferred because that concentration matches the appromimate oxygen content capacity of whole blood.

Among the not substantially surface active and not significantly water soluble oils that are useful in the emulsions and processes of this invention are liquid fatty oils, hydrocarbons, waxes, such as monoesters of a fatty acid and a monohydroxide alcohol, long chain ethers, diglycerides, silicone oils and nitriles. These include, for example, palmitoyl oleate, octyl nitrile, dodecyl nitrile, soy oil, safflower oil, hexadecane, diglycerides having a C₁₂₋₁₈ carbon chain and one unsaturation, and mineral oil. As with the fluoro-containing component, these oils also may be used singly or in various combinations in the emulsions and processes of this invention When our emulsions are to be used medically, the oil or combination of oils must, of course, be physiologically acceptable. For example, when our emulsions are to be used as "artificial bloods", we preferably use physiologically acceptable liquid fatty oils.

The amount of oil, or oils, present in the emulsions of this invention may vary over a wide range of concentrations. It depends on the concentration and properties of the other components of the emulsion, being principally dependent on the characteristics of the fluorocarbon component of the emulsion. The actual oil concentration to produce an acceptable emulsion for any given set of components is easily determined as taught by this invention using simple techniques of preparing and testing the stability of emulsions at various oil concentrations. Within this teaching, we typically employ between about 10 and 30% (by weight of the remaining non-fluorocarbon volume of the emulsion) of oil or a mixture of oils. Preferably, we employ between about 15 and 20% by weight.

Among the surfactants useful in the emulsions of this invention are any of the known anionic, cationic, non-ionic and zwitter-ionic surfactants. These include, for example, anionic surfactants, such as alkyl or aryl sulfates, sulfonates, carboxylates or phosphates, cationic surfactants such as mono-, di-, tri-, and tetraalkyl or aryl ammonium salts, non-ionic surfactants, such as alkyl or aryl compounds, whose hydrophilic part consists of polyoxyethylene chains, sugar molecules, polyalcohol derivatives or other hydrophilic groups and zwitterionic surfactants that may be combinations of the above anionic or cationic groups, and whose hydrophobic part consists of any other polymer, such as polyisobutylene or polypropylene oxides. Again, combinations of these surfactants may, of course, be used in the emulsions of this invention. In addition, mixtures of compounds, one or more of which are not surfactants, but which compounds when combined act as surfactants may also be usefully employed as the surfactant component of the emulsions of this invention.

Again, when the emulsions of this invention are to be used in "artificial bloods" or red blood cell substitutes, the surfactant, or combinations of them, must be physiologically acceptable. For example, in "artificial bloods" we prefer non-ionic surfactants. Preferably, the surfactants used in the emulsions of this invention are one or more of the following: egg phosphatides, lecithin, and alkyl salts of oleic acid, such as sodium oleate.

While the amount of a particular surfactant used in the emulsions of this invention depends on the amounts and properties of the other components of the emulsion, typically we employ about 0.5 to 7% (by weight of the non-fluorocarbon volume) of surfactant. More preferably, we use about 1-2% (by weight).

In addition to the highly fluorinated organic compounds, oils, surfactants and water, the emulsions of this invention may also contain other components conventionally used in "artificial bloods" or blood substitutes, oxygen transport agents or contrast agents for biological imaging. For example, when used as a blood substitute, an emulsion according to this invention should contain an isotonic agent, typically glycerol, to adjust the osmotic pressure of the emulsion to about that of blood. Typically we use about 2.5% (by weight of the non-fluorocarbon volume) of glycerol. However, other amounts and other osmotic pressure controlling agents, e.g., Tyrode solution, could as well be used. The emulsions of this invention may also include other components, such as oncotic agents, e.g., dextran or HES, and antioxidants.

The emulsions of this invention may be prepared using any order of mixing the four main components of our emulsions - highly fluorinated organic compound, oil, surfactant and water. However, for an optimal emulsion we prefer to mix the fluorocarbon first with the oil in the presence of a combination of all or part of the surfactant and some water. We then prepare the final emulsion by emulsifying this first emulsion in the remaining water and any remaining surfactant.

The mixing and emulsification of our components may be done using any of the conventional mixers and emulsifiers. For example, we may employ Fisher brand touch mixers and Microfluidizers. We may also, if desired, reduce the size of the average droplets or particles in our emulsions by conventional grinding.

In one form of the invention, for example, perfluoroindane is emulsified in water with suitable surfactant. In another form, perfluoroindane is emulsified in a previously prepared intravenous emulsion. Emulsions containing from about 20 to about 50% or more of the perfluoroindane by volume in water may be prepared. Emulsifying agents are included in an amount of about 1 to 10%, exemplified by egg phosphatide, phospholipid or polyoxyethylene-polyoxypropylene copolymer having molecular weight of about 8200 (Pluronic F-68). Other types of surface active agents may be used, including, albumin, glycerol, dextrans, gelatin or other naturally occuring surfactants. The non-ionic surfactants, which do not cause haemolysis are preferred. In addition to the perfluoroindane and surface active agent in the emulsion, it is preferred, but not essential, that the emulsion include an ionic component. Because most of the surfactants have sizeable osmotic activity, some consideration has to be given to modifying the ionic composition so as not to have a hypertonic solution. Generally speaking, in preparation of emulsions, there is added approximately 0.6 to 0.9% by weight sodium chloride since this is a concentration which can be infused rather rapidly without causing hemolysis. Ringer solution as is or diluted to half-strength with water is suitably used. Oxygen transport is facilitated in the coronary artery by introduction of perfluoroindane or liquid compositions containing perfluoroindane. An isolated organ of an animal may be perfused with perfluoroindane or liquid composition containing same. Furthermore, the perfluoroindane compositions of this invention may be employed utilizing NMR techniques according to procedures reported in U.S. Patent No. 4,586,511.

The following non-limiting examples illustrate various embodiments of this invention.

### Example 1

In this example, we prepared two emulsions in the same manner to compare their stabilities. The first emulsion was a conventional composition comprising 40% by volume perfluorodecalin and 60% by volume of a mixture of water (96.3% by weight), lecithin (1.2% by weight), glycerol (2.5% by weight) and sodium hydroxide to pH 8. The second emulsion was prepared according to this invention. It had the following composition: 40% by volume perfluorodecalin and 60% by volume of a mixture of water (78.8% by weight), lecithin (1.2% by weight), glycerol (2.5% by weight), soy oil (17.5% by weight) and sodium hydroxide to pH 8. The two emulsions were prepared by mixing their components together in a Fisher brand touch mixer and then running them through a Microfluidizer for 30 min at 4.14 bar (60 psi).

The first emulsion had a smaller average droplet size by optical microscopy than the second emulsion. It also had a higher concentration of water and thus less dispersed phase than the second emulsion. Accordingly, on those bases alone, we would have expected the first emulsion to be more stable than the second emulsion. However, the first "emulsion" was very unstable and exhibited phase separation at room temperature within 24 hours. The second emulsion (that prepared in accordance with this invention), while having a larger average droplet or particle size and more dispersed phase, was surprisingly very stable and showed substantially no phase separation and substantially no change in droplet or particle size distribution during 4 weeks storage at room temperature.

This comparison plainly demonstrates that the emulsions of this invention are different in kind from former compositions of highly fluorinated organic compounds. Not only are our emulsions far more stable, they are surprisingly more stable even with larger average particle or droplet size and more dispersed phase.

### Example 2

We prepared an emulsion containing 40% by volume perfluorodecalin and 60% by volume of a first emulsion containing safflower oil (10% by weight), soybean oil (10% by weight), lecithin (1.2% by weight), glycerol (2.5% by weight), water (76.3% by weight) and sodium hydroxide to pH 8.3. We prepared the final emulsion by combining 20 ml perfluorodecalin and 30 ml of the first emulsion and mixing the combination in a Fisher touch-mixer for 20 min. We then ran the resulting emulsion through a Microfluidizer for 1 hour at 4.14 bar (60 psi).

The resulting homogenized emulsion was still stable after 4 weeks at room temperature, as demonstrated by optical microscopy which indicated that there had been substantially no change in particle or droplet size distribution and substantially no phase separation.

### Example 3

Using the substantially same process as described in Example 2, we prepared an emulsion containing 40% by volume perfluorodecalin and 60% by volume of a first emulsion containing safflower oil (10% by weight), soybean oil (10% by weight), lecithin (2.0% by weight), glycerol (2.5% by weight), water (75.5% by weight) and sodium hydroxide to pH 8.3. As before, the resulting emulsion was still stable after 4 weeks at room temperature.

### Example 4

We prepared an emulsion containing 40% by volume perfluorodecalin and 60% by volume of a first emulsion containing safflower oil (10% by weight), soybean oil (10% by weight), lecithin (2.0% by weight), glycerol (2.5% by weight), XMO-20 (see, e.g., United States patent 4,443,480) (0.1% by weight), water (75.4% by weight) and sodium hydroxide to pH 8.3. We prepared the final emulsion by combining 20 ml perfluorodecalin and 30 ml of the first emulsion and mixing the combination in a Fisher touch mixer until the lecithin and XMO-20 were completely dissolved. We then ran the emulsion through a Microfluidizer for 30 min at 4.14 bar (60 psi). The resulting emulsion was still stable after 4 weeks at room temperature.

### Example 5

We prepared an emulsion containing 40% by volume perfluorodecalin and 60% by volume of a first emulsion containing safflower oil (10% by weight), soybean oil (10% by weight), lecithin (1.2% by weight), glycerol (2.5% by weight), oleic acid (0.8% by weight), water (75.5% by weight) and sodium hydroxide to pH 8.3. We prepared the final emulsion by mixing 20 ml perfluorodecalin and 30 ml of the first emulsion in a Fisher touch mixer for 10 min and running the resulting emulsion through a Microfluidizer for 45 min. The resulting emulsion was still stable after 4 weeks at room temperature.

### Example 6

We added lecithin to a final concentration of 2% (by weight) to the final emulsion of Example 5 and mixed it until the lecithin had completely dissolved. The emulsion gas then run through a Microfluidizer for 30 min at 4.14 bar (60 psi). The resulting emulsion was still stable after 4 weeks at room temperature.

### Example 7

We prepared an emulsion containing 40% by volume perfluorodecalin and 60% by volume of a mixture containing water (78.8% by weight), lecithin (1.2% by weight), glycerol (2.5% by weight), soybean oil (17.5% by weight) and sodium hydroxide to pH 8.0. We prepared the emulsion by mixing 0.377 g lecithin and 20 ml perfluorodecalin in a Fisher brand touch mixer for 10 min. We then added 5.4915 g soybean oil and mixed again for 10 min and added 0.7945 g glycerol and mixed again for 10 min. Finally, we added 24.721 g water stepwise with mixing. We made this addition by first adding 12.36 g of water to the mixture to disperse the fluorocarbon-oil-lecithin mixture and emulsified the resulting dispersion in a Microfluidizer for 30 min at 4.14 bar (60 psi). We then emptied the emulsion from the Microfluidizer and poured the remaining water into the Microfluidizer. After adding the previously prepared emulsion dropwise to the water, we ran the resulting mixture through the Microfluidizer for 30 min at 4.14 bar (60 psi) and adjusted the pH to 8.0 with sodium hydroxide. We then again ran the emulsion through a Microfluidizer for 30 min at 4.14 bar (60 psi). The final emulsion was still stable after 4 weeks at room temperature.

### Example 8

We prepared an emulsion containing 40% by volume perfluorodecalin and 60% by volume of a mixture containing water (78.8% by weight), lecithin (1.2% by weight), glycerol (2.5% by weight), hexadecane (17.5% by weight) and sodium hydroxide to pH 8.0. We used substantially the same method described in Example 7. The final emulsion was still stable after 4 weeks at room temperature.

### Example 9

We prepared an emulsion similar to that of Example 8, except that hexadecane was replaced with mineral oil. Again, we used substantially the same method described in Example 7 to prepare the emulsion. The final emulsion was still stable after 4 weeks at room temperature.

### Example 10

We prepared an emulsion containing 55% by volume perfluorodecalin and 45% by volume of a mixture containing safflower oil (10% by weight), soybean oil (10% by weight), glycerol (2.5% by weight), lecithin (2% by weight) and water (75.5% by weight) and sodium hydroxide to pH 8. To prepare the emulsion we used 18 ml of the oil-containing mixture, 22 ml of perfluorodecalin and 1% (by weight on total) oleic acid. We used the method substantially as described in Example 7. We mixed the final emulsion for 20 min in a Fisher touch mixer and then in a Microfluidizer for 15 cycles at 4.48 (65 psi). The emulsion was stable at room temperature.

### Example 11

We prepared an emulsion containing 70% by volume perfluorodecalin and 30% by volume of a mixture containing safflower oil (10% by weight), soybean oil (10% by weight), lecithin (1.2% by weight), glycerol (2.5% by weight), water (76.3% by weight) and sodium hydroxide to pH 8.0. To prepare the final emulsion we used 21 ml perfluorodecalin, 9 ml of the oil-containing mixture, and 0.5% (by weight on total) oleic acid. As in Example 10, we used substantially the same method described in Example 7 to prepare the final emulsion. We then mixed the final emulsion for 20 min in a Fisher touch mixer and then in a Microfluidizer for 15 cycles at 4.48 bar (65 psi). The emulsion was stable at room temperature.

### Example 12

We prepared an emulsion containing 16.5 ml perfluorooctylbromide (55% by volume) and 13.5 ml of the same mixture of other components described in Example 11. We used the same mixing and fluidizing regime described in Examples 10 and 11. The final emulsion was stable at room temperature.

### Example 13

An emulsion of perfluoro-cis-indane was prepared by mixing 10% by volume of the perfluoroindane in an intravenous fat emulsion, i.e., LIPOSYN II, manufactured by Abbott Laboratories. The LIPOSYN II is supplied in 10 and 20% concentrations. In this example, 10% concentration of LIPOSYN II is employed and contains 5% safflower oil, 5% soybean oil, up to 1.2% egg phosphatides added as an emulsifier and 2.5% glycerin in water for injection. Sodium hydroxide has been added to adjust the pH to approximately 8.0 LIPOSYN II 10% has an osmolarity of 320 mOsm/liter (approximate). The total caloric value of LIPOSYN II 10% including fat, phospholipid and glycerol is 1.1 kcal/ml. Of this total, approximately 0.6 kcal/ml is supplied by linoleic acid.

The perfluoroindane emulsion in this example was then injected into mice at a dose of 20cc/kilogram of body weight intravenously. Upon intravenous injection, there were no signs of toxicity and no evidence of gas or vapor embolism. It was also determined that the perfluoroindane left the animal body about five times faster than perfluorodecalin. More specifically, the average transpiration rate in microliters per day for perfluoroindane decreased from about 30 microliters/day upon infusion to a level of about 5 microliters/day at day 2, followed by rapid diminishment to less than 1 microliter/day after day 3.

By comparison, perfluoro-1,3,5-trimethylcyclohexane was emulsified in a similar fashion at a level of about 10% by volume within 10% LIPOSYN II and injected into mice at a dose level of about 20cc/kilogram intravenously. The average transpiration rate in microliters/day was observed at a level of about 10 immediately after infusion to a level of about 5 microliters/day after day 1, and about 2½ microliters/day after day 4. By comparison, therefore, the transpiration rate of perfluoroindane is considerably faster than the rate for perfluorotrimethylcyclohexane which is considered an analog or sister compound.

In a similar fashion, perfluoroisopropylcyclohexane was emulsified at a level of about 10% by volume with 10% LIPOSYN II and mice were injected at a dose of about 20cc/kilogram intravenously. As expected, the transpiration rate of the perfluoroisopropylcyclohexane was essentially the same as that for perfluorotrimethylcyclohexane. However, by comparison perfluoroindane has an unexpectedly faster rate of transpiration or elimination from the body of the mouse.

This example thus demonstrates that perfluoroindane may be injected into the animal body without causing gas or vapor embolism. Moreover, emulsions of perfluoroindane may be made and infused intravenously to function in a gas transport manner without toxic effects. Furthermore, upon comparison with analogous compounds, quite unexpectedly, the transpiration rate is significantly faster.

### Example 14

The procedures of Example 13 for perfluoro-cis-indane were repeated except that mice were injected with a dose of 40cc/kilogram intravenously of the 10% by volume emulsion with 10% LIPOSYN II. Similar transpiration rates were observed with the larger dose of perfluoroindane except that larger amounts of perfluoroindane were being transpired post injection, namely, about 30 microliters/day at about day 1, about 17 microliters/day at day 2, about 10 microliters/day at day 3, about 4 microliters/day at day 4 and nearly 0 microliters/day at day 7. Otherwise, the experiments with mice injected at a level of 40cc/kilogram intravenously produced essentially the same results as those at the lower dose of 20cc/kilogram according to Example 13.

The reader's attention is drawn to EP-A-0220153 (Application No. 88850373.1) which is comprised in the state of the art within the meaning of Article 54(3) EPC for the Contracting States DE, ES, FR, IT and SE and which discloses oxygen-transporting emulsions which comprise, amongst other components, from 5-50 g per 100 ml of emulsion of a perfluorocarbon compound.

## Claims (Claims for the following Contracting State(s): AT, BE, CH, GR, LI, LU, NL)

1. A physiologically acceptable emulsion comprising a highly fluorinated organic compound; an oil that is not substantially surface active and not significantly water soluble; a surfactant; and water.

2. The emulsion according to claim 1, wherein the highly fluorinated organic compound is present in an amount up to 75 %, by volume.

3. The emulsion according to claim 1, wherein the highly fluorinated organic compound is present in an amount from 10 to 70 %, by volume.

4. The emulsion according to any preceding claim, wherein the highly fluorinated organic compound is selected from perfluorodecalin, perfluorodimethyladamantane, perfluorooctylbromide, perfluoro-4-methyl-octahydoquinolidizine, perfluoro-N-methyl-decahydroquinoline, F-methyl-1-oxa-decalin, perfluoro-bicyclo[5.3. 0]decane, perfluorooctahydroquinolidizine, perfluoro-5,6-dihydro-5-decene, perfluoroindane, perfluorotrimethylcyclohexane, perfluoroisopropylcyclohexane and perfluoro-4,5-dihydro-4-octene.

5. The emulsion according to any preceding claim, wherein the emulsion is stable after heating at about 115°C for about 15 minutes.

6. The emulsion according to any preceding claim, wherein the oil is present in an amount from 10 to 30 %, by weight of the non-highly fluorinated organic compound volume.

7. The emulsion according to any preceding claim, wherein the oil is present in an amount from 15 to 20 %, by weight of the non-highly fluorinated organic compound volume.

8. The emulsion according to any preceding claim, wherein the oil is a liquid fatty oil.

9. The emulsion according to any preceding claim, wherein the surfactant is present in an amount from 0.5 to 7 %, by weight of the non-highly fluorinated organic compound volume.

10. The emulsion according to any preceding claim, wherein the surfactant is present in an amount from 1 to 2 %, by weight of the non-highly fluorinated organic compound volume.

11. The emulsion according to any preceding claim, wherein the surfactant is selected from lecithin, egg phosphatides and alkyl salts of oleic acid.

12. The emulsion according to any preceding claim, wherein the emulsion further comprises at least one compound selected from oncotic agents, osmotic pressure controlling agents, serum extending agents and antioxidants.

13. A red blood cell substitute comprising an amount of an emulsion according to any one of claims 1-12, which amount is therapeutically effective for oxygen carrying and transport in animals, including humans.

14. A contrast agent for biological imaging comprising an amount of an emulsion according to any one of claims 1-12, which amount is clinically effective for imaging by a modality selected from nuclear magnetic resonance, x-ray and ultrasound.

15. The emulsion according to any one of claims 1-12, for introduction into an animal body or isolated animal organ for supporting oxygen transport therein.

16. A method of preparing an emulsion according to any one of claims 1-12, said method comprising the steps of preparing a first emulsion by mixing the highly fluorinated organic compound with the oil in the presence of all or a portion of the surfactant and some of the water, and preparing a final emulsion by emulsifying the first emulsion, the remaining water and any remaining surfactant.

17. The method according to claim 16, further comrising the step of sterilizing the final emulsion by heating it to about 115°C, said emulsion maintaining its stability under those conditions.

## Claims (Claims for the following Contracting State(s): DE, FR, IT, SE)

1. A physiologically acceptable emulsion comprising a highly fluorinated organic compound; an oil that is not substantially surface active and not significantly water soluble; a surfactant; and water; with the proviso that the emulsion does not contain from 5-50g of said highly fluorinated organic compound per 100ml of the emulsion.

2. The emulsion according to claim 1, wherein the surfactant is present in an amount from 0.5 to 7 %, by weight of the non highly fluorinated organic compound volume.

3. The emulsion according to claim 1, wherein the highly fluorinated organic compound is present in an amount upto 75 %, by volume.

4. The emulsion according to any preceding claim, wherein the highly fluorinated organic compound is selected from perfluorodecalin, perfluorodimethyladamantane, perfluorooctylbromide, perfluoro-4-methyl-octahydoquinolidizine, perfluoro-N-methyl-decahydroquinoline, F-methyl-1-oxa-decalin, perfluoro-bicyclo[5.3.0]decane, perfluoro-octahydroquinolidizine, perfluoro-5,6-dihydro-5-decene, perfluoroindane, perfluorotrimethylcyclohexane, perfluoroisopropylcyclohexane and perfluoro-4,5-dihydro-4-octene.

5. The emulsion according to any preceding claim, wherein the emulsion is stable after heating at about 115°C for about 15 minutes.

6. The emulsion according to any preceding claim, wherein the oil is present in an amount from 10 to 30 %, by weight of the non-highly fluorinated organic compound volume.

7. The emulsion according to any preceding claim, wherein the oil is present in an amount from 15 to 20 %, by weight of the non-highly fluorinated organic compound volume.

8. The emulsion according to any preceding claim, wherein the oil is a liquid fatty oil.

9. The emulsion according to any preceding claim, wherein the surfactant is present in an amount from 0.5 to 7 %, by weight of the non-highly fluorinated organic compound volume.

10. The emulsion according to any preceding claim, wherein the surfactant is present in an amount from 1 to 2 %, by weight of the non-highly fluorinated organic compound volume.

11. The emulsion according to any preceding claim, wherein the surfactant is selected from lecithin, egg phosphatides and alkyl salts of oleic acid.

12. The emulsion according to any preceding claim, wherein the emulsion further comprises at least one compound selected from oncotic agents, osmotic pressure controlling agents, serum extending agents and antioxidants.

13. A red blood cell substitute comprising an amount of an emulsion according to any one of claims 1-12, which amount is therapeutically effective for oxygen carrying and transport in animals, including humans.

14. A contrast agent for biological imaging comprising an amount of an emulsion according to any one of claims 1-12, which amount is clinically effective for imaging by a modality selected from nuclear magnetic resonance, x-ray and ultrasound.

15. The emulsion according to any one of claims 1-12, for introduction into an animal body or isolated animal organ for supporting oxygen transport therein.

16. A method of preparing an emulsion according to any one of claims 1-12, said method comprising the steps of preparing a first emulsion by mixing the highly fluorinated organic compound with the oil in the presence of all or a portion of the surfactant and some of the water, and preparing a final emulsion by emulsifying the first emulsion, the remaining water and any remaining surfactant.

17. The method according to claim 16, further comrising the step of sterilizing the final emulsion by heating it to about 115°C, said emulsion maintaining its stability under those conditions.

## Claims (Claims for the following Contracting State(s): ES)

1. A method of preparing a physiologically acceptable emulsion, characterised by the steps of:
(a) mixing in any order:
(i) a highly fluorinated organic compound,
(ii) an oil that is not substantially surface active and not significantly water soluble,
(iii) a surfactant, and
(iv) water, and
(b) recovering the resulting emulsion;
with the proviso that said emulsion which is recovered in step (b) does not contain from 5-50 g of said highly fluorinated organic compound per 100 ml of the emulsion.

2. A method according to Claim 1, wherein the surfactant is used in an amount from 0.5 to 7%, by weight of the non highly fluorinated organic compound volume.

3. A method according to Claim 1 wherein the highly fluorinated organic compound is used in an amount up to to 75%, by volume.

4. A method according to any preceding claim, wherein the highly fluorinated organic compound is selected from perfluorodecalin, perfluorodimethyladamantane, perfluorooctylbromide, perfluoro-4-methyloctahydroquinolidizine, perfluoro-N-methyl-decahydroquinoline, F-methyl-1-oxa-decalin, perfluoro-bicyclo[5.3.0]decane, perfluoro-octahydroquinolidizine, perfluoro-5,6-dihydro-5-decene, perfluoroindane, perfluorotrimethylcyclohexane, perfluoroisopropylcyclohexane and perfluoro-4,5-dihydro-4-octene.

5. A method according to any preceding claim, wherein there is produced an emulsion which is stable after heating at about 115°C for about 15 minutes.

6. A method according to any preceding claim, wherein the oil is used in an amount from 10 to 30%, by weight of the non-highly fluorinated organic compound volume.

7. A method according to any preceding claim, wherein the oil is used in an amount between about 15 and 20%, by weight of the non-highly fluorinated organic compound volume.

8. A method according to any preceding claim, wherein the oil is a liquid fatty oil.

9. A method according to any preceding claim, wherein the surfactant is used in an amount from 0.5 to 7%, by weight of the non-highly fluorinated organic compound volume.

10. A method according to any preceding claim, wherein the surfactant is used in an amount from 1 to 2%, by weight of the non-highly fluorinated organic compound volume.

11. A method according to any preceding claim, wherein the surfactant is selected from lecithin, egg phosphatides and alkyl salts of oleic acid.

12. A method according to any preceding claim, wherein the mixture formed in step (a) further comprises at least one compound selected from oncotic agents, osmotic pressure controlling agents, serum extending agents and antioxidants.

13. A method according to any preceding claim, comprising the steps of preparing a first emulsion by mixing the highly fluorinated organic compound with the oil in the presence of all or a portion of the surfactant and some of the water, and preparing a final emulsion by emulsifying the first emulsion, the remaining water and any remaining surfactant.

14. A method according to any preceding claim, further comprising the step of sterilizing the emulsion recovered in step (b) by heating it to about 115°C, said emulsion maintaining its stability under those conditions.

## Patentansprüche (Patentansprüche für folgende(n) Vertragsstaat(en): AT, BE, CH, GR, LI, LU, NL)

1. Physiologisch verträgliche Emulsion, die eine hochfluorierte organische Verbindung; ein Öl, das nicht wesentlich oberflächenaktiv und nicht wesentlich wasserlöslich ist; ein Tensid; und Wasser enthält.

2. Emulsion nach Anspruch 1, worin die hochfluorierte organische Verbindung in einer Menge von bis zu 75 Vol.-% vorhanden ist.

3. Emulsion nach Anspruch 1, worin die hochfluorierte organische Verbindung in einer Menge von 10 bis 70 Vol.-% vorhanden ist.

4. Emulsion nach einem der vorhergehenden Ansprüche, worin die hochfluorierte organische Verbindung aus Perfluordecalin, Perfluordimethyladamantan, Perfluoroctylbromid, Perfluor-4-methyloctahydrochinolidizin, Perfluor-N-methyldecahydrochinolin, F-Methyl-1-oxadecalin, Perfluorbizyklo[5.3.0]decan, Perfluoroctahydrochinolidizin, Perfluor-5,6-dihydro-5-decen, Perfluorindan, Perfluortrimethylzyklohexan, Perfluorisopropylzyklohexan und Perfluor-4,5-dihydro-4-octen ausgewählt ist.

5. Emulsion nach einem der vorhergehenden Ansprüche, worin die Emulsion nach etwa 15-minütigem Erwärmen auf etwa 115°C stabil ist.

6. Emulsion nach einem der vorhergehenden Ansprüche, worin das Öl in einer Menge von 10 bis 30 Gew.-%, bezogen auf das Volumen, das nicht von der hochfluorierten organischen Verbindung eingenommen wird, vorhanden ist.

7. Emulsion nach einem der vorhergehenden Ansprüche, worin das Öl in einer Menge von 15 bis 20 Gew.-%, bezogen auf das Volumen, das nicht von der hochfluorierten organischen Verbindung eingenommen wird, vorhanden ist.

8. Emulsion nach einem der vorhergehenden Ansprüche, worin das Öl ein flüssiges fettes Öl ist.

9. Emulsion nach einem der vorhergehenden Ansprüche, worin das Tensid in einer Menge von 0,5 bis 7 Gew.-%, bezogen auf das Volumen, das nicht von der hochfluorierten organischen Verbindung eingenommen wird, vorhanden ist.

10. Emulsion nach einem der vorhergehenden Ansprüche, worin das Tensid in einer Menge von 1 bis 2 Gew.-%, bezogen auf das Volumen, das nicht von der hochfluorierten organischen Verbindung eingenommen wird, vorhanden ist.

11. Emulsion nach einem der vorhergehenden Ansprüche, worin das Tensid aus Lecithin, Eiphosphatiden und Alkylsalzen von Ölsäure ausgewählt ist.

12. Emulsion nach einem der vorhergehenden Ansprüche, worin die Emulsion weiters zumindest eine Verbindung enthält, die aus oncotischen Mitteln, den osmotischen Druck steuernden Mitteln, serumstreckenden Mitteln und Oxidationshemmern ausgewählt sind.

13. Ersatzmittel für rote Blutkörperchen, das eine Menge einer Emulsion nach einem der Ansprüche 1-12 umfaßt, welche Menge zum Tragen und Transportieren von Sauerstoff in Tieren, einschließlich des Menschen, therapeutisch wirksam ist.

14. Kontrastmittel zum biologischen Abbilden, das eine Menge einer Emulsion nach einem der Ansprüche 1-12 umfaßt, welche Menge klinisch wirksam zum Abbilden durch eine Modalität ist, die aus nuklearmagnetischer Resonanz, Röntgenstrahlen und Ultraschall ausgewählt ist.

15. Emulsion nach einem der Ansprüche 1-12 zum Einbringen in einen Tierkörper oder ein isoliertes Tierorgan zum Unterstützen des Sauerstofftransports darin.

16. Verfahren zum Herstellen einer Emulsion nach einem der Ansprüche 1-12, wobei das genannte Verfahren die Schritte des Herstellens einer ersten Emulsion durch Mischen der hochfluorierten organischen Verbindung mit dem Öl in der Gegenwart des gesamten oder einer Teilmenge des Tensids und eines Teils des Wassers, und des Herstellens einer Endemulsion durch Emulgieren der ersten Emulsion, des verbleibenden Wassers und jeglichen verbleibenden Tensids umfaßt.

17. Verfahren nach Anspruch 16, das weiters den Schritt des Sterilisierens der Endemulsion durch ihr Erwärmen auf etwa 115°C umfaßt, wobei die genannte Emulsion ihre Stabilität unter diesen Bedingungen beibehält.

## Patentansprüche (Patentansprüche für folgende(n) Vertragsstaat(en): DE, FR, IT, SE)

1. Physiologisch verträgliche Emulsion, die eine hochfluorierte organische Verbindung; ein Öl, das nicht wesentlich oberflächenaktiv und nicht wesentlich wasserlöslich ist; ein Tensid; und Wasser enthält; unter der Voraussetzung, daß die Emulsion nicht 5-50 g der genannten hochfluorierten organischen Verbindung pro 100 ml der Emulsion enthält.

2. Emulsion nach Anspruch 1, worin das Tensid in einer Menge von 0,5 bis 7 Gew.% bezogen auf das Volumen, das nicht von der hochfluorierten organischen Verbindung eingenommen wird, vorhanden ist.

3. Emulsion nach Anspruch 1, worin die hochfluorierte organische Verbindung in einer Menge von bis zu 75 Vol.-% vorhanden ist.

4. Emulsion nach einem der vorhergehenden Ansprüche, worin die hochfluorierte organische Verbindung aus Perfluordecalin, Perfluordimethyladamantan, Perfluoroctylbromid, Perfluor-4-methyloctahydrochinolidizin, Perfluor-N-methyldecahydrochinolin, F-Methyl-1-oxadecalin, Perfluorbizyklo[5.3.0]decan, Perfluoroctahydrochinolidizin, Perfluor-5,6-dihydro-5-decen, Perfluorindan, Perfluortrimethylzyklohexan, Perfluorisopropylzyklohexan und Perfluor-4,5-dihydro-4-octen ausgewählt ist.

5. Emulsion nach einem der vorhergehenden Ansprüche, worin die Emulsion nach etwa 15-minütigem Erwärmen auf etwa 115°C stabil ist.

6. Emulsion nach einem der vorhergehenden Ansprüche, worin das Öl in einer Menge von 10 bis 30 Gew.-%, bezogen auf das Volumen, das nicht von der hochfluorierten organischen Verbindung eingenommen wird, vorhanden ist.

7. Emulsion nach einem der vorhergehenden Ansprüche, worin das Öl in einer Menge von 15 bis 20 Gew.-%, bezogen auf das Volumen, das nicht von der hochfluorierten organischen Verbindung eingenommen wird, vorhanden ist.

8. Emulsion nach einem der vorhergehenden Ansprüche, worin das Öl ein flüssiges fettes Öl ist.

9. Emulsion nach einem der vorhergehenden Ansprüche, worin das Tensid in einer Menge von 0,5 bis 7 Gew.-%, bezogen auf das Volumen, das nicht von der hochfluorierten organischen Verbindung eingenommen wird, vorhanden ist.

10. Emulsion nach einem der vorhergehenden Ansprüche, worin das Tensid in einer Menge von 1 bis 2 Gew.-%, bezogen auf das Volumen, das nicht von der hochfluorierten organischen Verbindung eingenommen wird, vorhanden ist.

11. Emulsion nach einem der vorhergehenden Ansprüche, worin das Tensid aus Lecithin, Eiphosphatiden und Alkylsalzen von Ölsäure ausgewählt ist.

12. Emulsion nach einem der vorhergehenden Ansprüche, worin die Emulsion weiters zumindest eine Verbindung enthält, die aus oncotischen Mitteln, den osmotischen Druck steuernden Mitteln, serumstreckenden Mitteln und Oxidationshemmern ausgewählt sind.

13. Ersatzmittel für rote Blutkörperchen, das eine Menge einer Emulsion nach einem der Ansprüche 1-12 umfaßt, welche Menge zum Tragen und Transportieren von Sauerstoff in Tieren, einschließlich des Menschen, therapeutisch wirksam ist.

14. Kontrastmittel zum biologischen Abbilden, das eine Menge einer Emulsion nach einem der Ansprüche 1-12 umfaßt, welche Menge klinisch wirksam zum Abbilden durch eine Modalität ist, die aus kernmagnetischer Resonanz, Röntgenstrahlen und Ultraschall ausgewählt ist.

15. Emulsion nach einem der Ansprüche 1-12 zum Einbringen in einen Tierkörper oder ein isoliertes Tierorgan zum Unterstützen des Sauerstofftransports darin.

16. Verfahren zum Herstellen einer Emulsion nach einem der Ansprüche 1-12, wobei das genannte Verfahren die Schritte des Herstellens einer ersten Emulsion durch Mischen der hochfluorierten organischen Verbindung mit dem Öl in der Gegenwart des gesamten oder einer Teilmenge des Tensids und eines Teils des Wassers, und des Herstellens einer Endemulsion durch Emulgieren der ersten Emulsion, des verbleibenden Wassers und jeglichen verbleibenden Tensids umfaßt.

17. Verfahren nach Anspruch 16, das weiters den Schritt des Sterilisierens der Endemulsion durch ihr Erwärmen auf etwa 115°C umfaßt, wobei die genannte Emulsion ihre Stabilität unter diesen Bedingungen beibehält.

## Patentansprüche (Patentansprüche für folgende(n) Vertragsstaat(en): ES)

1. Verfahren zum Herstellen einer physiologisch verträglichen Emulsion, gekennzeichnet durch die Schritte des:
(a) Mischens in jeglicher Reihenfolge von:
(i) einer hochfluorierten organischen Verbindung;
(ii) einem Öl, das nicht wesentlich oberflächenaktiv und nicht wesentlich wasserlöslich ist;
(iii) einem Tensid; und
(iv)Wasser, und
(b) Gewinnens der resultierenden Emulsion;
unter der Voraussetzung, daß die genannte Emulsion, die in Schritt (b) gewonnen wird, nicht von 5-50 g der genannten hochfluorierten organischen Verbidnung pro 100 ml der Emulsion enthält.

2. Verfahren nach Anspruch 1, worin das Tensid in einer Menge von 0,5 bis 7 Gew.-%, bezogen auf das Volumen, das nicht von der hochfluorierten organischen Verbindung eingenommen wird, verwendet wird.

3. Verfahren nach Anspruch 1, worin die hochfluorierte organische Verbindung in einer Menge von bis zu 75 Vol.-% verwendet wird.

4. Verfahren nach einem der vorhergehenden Ansprüche, worin die hochfluorierte organische Verbindung aus Perfluordecalin, Perfluordimethyladamantan, Perfluoroctylbromid, Perfluor-4-methyloctahydrochinolidizin, Perfluor-N-methyldecahydrochinolin, F-Methyl-1-oxadecalin, Perfluorbizyklo[5.3.0]decan, Perfluoroctahydrochinolidizin, Perfluor-5,6-dihydro-5-decen, Perfluorindan, Perfluortrimethylzyklohexan, Perfluorisopropylzyklohexan und Perfluor-4,5-dihydro-4-octen ausgewählt wird.

5. Verfahren nach einem der vorhergehenden Ansprüche, worin eine Emulsion erzeugt wird, die nach etwa 15-minütigem Erwärmen auf etwa 115°C stabil ist.

6. Verfahren nach einem der vorhergehenden Ansprüche, worin das Öl in einer Menge von 10 bis 30 Gew.-%, bezogen auf das Volumen, das nicht von der hochfluorierten organischen Verbindung eingenommen wird, verwendet wird.

7. Verfahren nach einem der vorhergehenden Ansprüche, worin das Öl in einer Menge zwischen etwa 15 bis 20 Gew.-%, bezogen auf das Volumen, das nicht von der hochfluorierten organischen Verbindung eingenommen wird, verwendet wird.

8. Emulsion nach einem der vorhergehenden Ansprüche, worin das Öl ein flüssiges fettes Öl ist.

9. Emulsion nach einem der vorhergehenden Ansprüche, worin das Tensid in einer Menge von 0,5 bis 7 Gew.-%, bezogen auf das Volumen, das nicht von der hochfluorierten organischen Verbindung eingenommen wird, verwendet wird.

10. Verfahren nach einem der vorhergehenden Ansprüche, worin das Tensid in einer Menge von 1 bis 2 Gew.-%, bezogen auf das Volumen, das nicht von der hochfluorierten organischen Verbindung eingenommen wird, verwendet wird.

11. Verfahren nach einem der vorhergehenden Ansprüche, worin das Tensid aus Lecithin, Eiphosphatiden und Alkylsalzen von Ölsäure ausgewählt wird.

12. Verfahren nach einem der vorhergehenden Ansprüche, worin die in Schritt (a) gebildetete Mischung weiters zumindest eine Verbindung enthält, die aus oncotischen Mitteln, den osmotischen Druck steuernden Mitteln, serumstreckenden Mitteln und Oxidationshemmern ausgewählt sind.

13. Verfahren nach einem der vorhergehenden Ansprüche, das die Schritte des Herstellens einer ersten Emulsion durch Mischen der hochfluorierten organischen Verbindung mit dem Öl in der Gegenwart des gesamten oder einer Teilmenge des Tensids und eines Teils des Wassers, und des Herstellens einer Endemulsion durch Emulgieren der ersten Emulsion, des verbleibenden Wassers und jeglichen verbleibenden Tensids umfaßt.

14. Verfahren nach einem der vorhergehenden Ansprüche, das weiters den Schritt des Sterilisierens der in Schritt (b) gewonnenen Endemulsion durch ihr Erwärmen auf etwa 115°C umfaßt, wobei die genannte Emulsion ihre Stabilität unter diesen Bedingungen beibehält.

## Revendications (Revendications pour l'(les) Etat(s) contractant(s) suivant(s): AT, BE, CH, GR, LI, LU, NL)

1. Emulsion physiologiquement acceptable comprenant un composé organique fortement fluoré; une huile qui n'est pas notablement tensio-active et qui n'est pas significativement soluble dans l'eau; un agent tensio-actif; et de l'eau.

2. Emulsion selon la revendication 1, dans laquelle le composé organique fortement fluoré est présent dans une proportion allant jusqu'à 75 % en volume.

3. Emulsion selon la revendication 1, dans laquelle le composé organique fortement fluoré est présent dans une proportion de 10 à 70 % en volume.

4. Emulsion selon l'une quelconque des revendications précédentes, dans laquelle le composé organique fortement fluoré est choisi parmi la perfluorodécaline, le perfluorodiméthyladamantane, le bromure de perfluoroctyle, la perfluoro-4-méthyl-octahydroquinolidizine, la perfluoro-N-méthyl-décahydroquinoléine, la F-méthyl-1-oxa-décaline, le perfluorobicyclo[5.3.0]décane, la perfluoro-octahydroquinolidizine, le perfluoro-5,6-dihydro-5-décène, le perfluorindane, le perfluorotriméthylcyclohexane, le perfluoroisopropylcyclohexane et le perfluoro-4,5-dihydro-4-octène.

5. Emulsion selon l'une quelconque des revendications précédentes, qui est stable après chauffage aux environs de 115°C pendant environ 15 minutes.

6. Emulsion selon l'une quelconque des revendications précédentes, dans laquelle l'huile est présente dans une proportion de 10 à 30 % en poids par rapport au volume des constituants autres que le composé organique fortement fluoré.

7. Emulsion selon l'une quelconque des revendications précédentes, dans laquelle l'huile est présente dans une proportion de 15 à 20 % en poids par rapport au volume des constituants autres que le composé organique fortement fluoré.

8. Emulsion selon l'une quelconque des revendications précédentes, dans laquelle l'huile est une huile grasse liquide.

9. Emulsion selon l'une quelconque des revendications précédentes, dans laquelle l'agent tensio-actif est présent dans une proportion de 0,5 à 7 % en poids par rapport au volume des constituants autres que le composé organique fortement fluoré.

10. Emulsion selon l'une quelconque des revendications précédentes, dans laquelle l'agent tensio-actif est présent dans une proportion de 1 à 2 % en poids par rapport au volume des constituants autres que le composé organique fortement fluoré.

11. Emulsion selon l'une quelconque des revendications précédentes, dans laquelle l'agent tensio-actif est choisi parmi la lécithine, les phosphatides d'oeuf et les sels d'alkyle de l'acide oléique.

12. Emulsion selon l'une quelconque des revendications précédentes, qui comprend en outre au moins un composé choisi parmi des agents oncotiques, des agents de réglage de la pression osmotique, des succédanés du sérum et des anti-oxydants.

13. Substitut des globules rouges comprenant une proportion de l'émulsion selon l'une quelconque des revendications 1 à 12, laquelle proportion est thérapeutiquement efficace pour se charger d'oxygène et de le transporter chez les animaux, y compris l'homme.

14. Agent de contraste pour la formation d'images biologiques comprenant une proportion d'une émulsion selon l'une quelconque des revendications 1 à 12, laquelle proportion est cliniquement efficace pour former une image par des techniques choisies parmi la résonance magnétique nucléaire, les rayons x et les ultra-sons.

15. Emulsion selon l'une quelconque des revendications 1 à 12, pour l'introduction dans l'organisme d'un animal ou dans un organe d'animal isolé pour servir de support au transport de l'oxygène dans celui-ci.

16. Procédé de préparation d'une émulsion selon l'une quelconque des revendications 1 à 12, ce procédé comprenant les stades consistant à préparer une première émulsion en mélangeant le composé organique fortement fluoré avec l'huile en présence de tout ou partie de l'agent tensio-actif et d'une partie de l'eau, et à préparer une émulsion finale en émulsionnant la première émulsion, le reste de l'eau et l'agent tensio-actif éventuellement restant.

17. Procédé selon la revendication 16, comprenant en outre le stade consistant à stériliser l'émulsion finale en la chauffant aux environs de 115°C, cette émulsion conservant sa stabilité dans ces conditions.

## Revendications (Revendications pour l'(les) Etat(s) contractant(s) suivant(s): DE, FR, IT, SE)

1. Emulsion physiologiquement acceptable comprenant un composé organique fortement fluoré; une huile qui n'est pas notablement tensio-active et qui n'est pas significativement soluble dans l'eau; un agent tensio-actif; et de l'eau; sous réserve que l'émulsion ne contient pas de 5 à 50 g de ce composé organique fortement fluoré pour 100 ml de l'émulsion.

2. Emulsion selon la revendication 1, dans laquelle l'agent tensio-actif est présent dans une proportion de 0,5 à 7 % en poids par rapport au volume du composé organique non fortement fluoré.

3. Emulsion selon la revendication 1, dans laquelle le composé organique fortement fluoré est présent dans une proportion allant jusqu'à 75 % en volume.

4. Emulsion selon l'une quelconque des revendications précédentes, dans laquelle le composé organique fortement fluoré est choisi parmi la perfluorodécaline, le perfluorodiméthyladamantane, le bromure de perfluoroctyle, la perfluoro-4-méthyloctahydroquinolidizine, la perfluoro-N-méthyl-décahydroquinoléine, la F-méthyl-1-oxa-décaline, le perfluorobicyclo[5.3.0]décane, la perfluoro-octahydroquinolidizine, le perfluoro-5,6-dihydro-5-décène, le perfluoroindane, le perfluorotriméthylcyclohexane, le perfluoroisopropylcyclohexane et le perfluoro-4,5-dihydro-4-octène.

5. Emulsion selon l'une quelconque des revendications précédentes, qui est stable après chauffage aux environs de 115°C pendant environ 15 minutes.

6. Emulsion selon l'une quelconque des revendications précédentes, dans laquelle l'huile est présente dans une proportion de 10 à 30 % en poids par rapport au volume des constituants autres que le composé organique fortement fluoré.

7. Emulsion selon l'une quelconque des revendications précédentes, dans laquelle l'huile est présente dans une proportion de 15 à 20 % en poids par rapport au volume des constituants autres que le composé organique fortement fluoré.

8. Emulsion selon l'une quelconque des revendications précédentes, dans laquelle l'huile est une huile grasse liquide.

9. Emulsion selon l'une quelconque des revendications précédentes, dans laquelle l'agent tensio-actif est présent dans une proportion de 0,5 à 7 % en poids par rapport au volume des constituants autres que le composé organique fortement fluoré.

10. Emulsion selon l'une quelconque des revendications précédentes, dans laquelle l'agent tensio-actif est présent dans une proportion de 1 à 2 % en poids par rapport au volume des constituants autres que le composé organique fortement fluoré.

11. Emulsion selon l'une quelconque des revendications précédentes, dans laquelle l'agent tensio-actif est choisi parmi la lécithine, les phosphatides d'oeuf et les sels d'alkyle de l'acide oléique.

12. Emulsion selon l'une quelconque des revendications précédentes, qui comprend en outre au moins un composé choisi parmi des agents oncotiques, des agents de réglage de la pression osmotique, des succédanés du sérum et des anti-oxydants.

13. Substitut des globules rouges comprenant une proportion d'une émulsion selon l'une quelconque des revendications 1 à 12, laquelle proportion est thérapeutiquement efficace pour qu'elle se charge d'oxygène et qu'elle le transporte chez les animaux, y compris l'homme.

14. Agent de contraste pour la formation d'images biologiques comprenant une proportion d'une émulsion selon l'une quelconque des revendications 1 à 12, laquelle proportion est cliniquement efficace pour former une image par des techniques choisies parmi la résonance magnétique nucléaire, les rayons x et les ultra-sons.

15. Emulsion selon l'une quelconque des revendications 1 à 12, pour l'introduction dans l'organisme d'un animal ou dans un organe d'animal isolé pour servir de support au transport de l'oxygène dans celui-ci.

16. Procédé de préparation d'une émulsion selon l'une quelconque des revendications 1 à 12, ce procédé comprenant les stades consistant à préparer une première émulsion en mélangeant le composé organique fortement fluoré avec l'huile en présence de tout ou partie de l'agent tensio-actif et d'une partie de l'eau, et à préparer une émulsion finale en émulsionnant la première émulsion, le reste de l'eau et l'agent tensio-actif éventuellement restant.

17. Procédé selon la revendication 16, comprenant en outre le stade consistant à stériliser l'émulsion finale en la chauffant aux environs de 115°C, cette émulsion conservant sa stabilité dans ces conditions.

## Revendications (Revendications pour l'(les) Etat(s) contractant(s) suivant(s): ES)

1. Procédé de préparation d'une émulsion physiologiquement acceptable, caractérisé par les stades consistant à :
(a) mélanger dans n'importe quel ordre :
(i) un composé organique fortement fluoré,
(ii) une huile qui n'est pas notablement tensio-active et n'est pas significativement soluble dans l'eau,
(iii) un agent tensio-actif, et
(iv) de l'eau, et
(b) à récupérer l'émulsion obtenue; sous réserve que cette émulsion qui est récupérée dans le stade (b) ne contient pas de 5 à 50 g de ce composé organique fortement fluoré pour 100 ml de l'émulsion.

2. Procédé selon la revendication 1, dans lequel l'agent tensio-actif est utilisé dans une proportion de 0,5 à 7 % en poids par rapport au volume des constituants autres que le composé organique non fortement fluoré.

3. Procédé selon la revendication 1, dans lequel le composé organique fortement fluoré est utilisé dans une proportion allant jusqu'à 75 % en volume.

4. Procédé selon l'une quelconque des revendications précédentes, dans lequel le composé organique fortement fluoré est choisi parmi la perfluorodécaline, le perfluorodiméthyladamantane, le bromure de perfluoroctyle, la perfluoro-4-méthyl-octahydroquinolidizine, la perfluoro-N-méthyl-décahydroquinoléine, la F-méthyl-1-oxa-décaline, le perfluoro-bicyclo[5.3.0]décane, la perfluoro-octahydroquinolidizine, le perfluoro-5,6-dihydro-5-décène, le perfluoroindane, le perfluorotriméthylcyclohexane, le perfluoroisopropylcyclohexane et le perfluoro-4,5-dihydro-4-octène.

5. Procédé selon l'une quelconque des revendications précédentes, dans lequel on prépare une émulsion qui est stable après chauffage à environ 115°C pendant environ 15 minutes.

6. Procédé selon l'une quelconque des revendications précédentes, dans lequel l'huile est utilisée dans une proportion de 10 à 30 % en poids par rapport au volume des constituants autres que le composé organique fortement fluoré.

7. Procédé selon l'une quelconque des revendications précédentes, dans lequel l'huile est utilisée dans une proportion comprise entre environ 15 et 20 % en poids par rapport au volume des constituants autres que le composé organique fortement fluoré.

8. Procédé selon l'une quelconque des revendications précédentes, dans lequel l'huile est une huile grasse liquide.

9. Procédé selon l'une quelconque des revendications précédentes, dans lequel l'agent tensio-actif est utilisé dans une proportion de 0,5 à 7 % en poids par rapport au volume des constituant autres que le composé organique fortement fluoré.

10. Procédé selon l'une quelconque des revendications précédentes, dans lequel l'agent tensio-actif est utilisé dans une proportion de 1 à 2 % en poids par rapport au volume des constituants autres que le composé organique fortement fluoré.

11. Procédé selon l'une quelconque des revendications précédentes, dans lequel l'agent tensio-actif est choisi parmi la lécithine, les phosphatides d'oeuf et les sels d'alkyle de l'acide oléique.

12. Procédé selon l'une quelconque des revendications précédentes, dans lequel le mélange formé dans le stade (a) comprend en outre au moins un composé choisi parmi des agents oncotiques, des agents de réglage de la pression osmotique, des succédanés du sérum et des anti-oxydants.

13. Procédé selon l'une quelconque des revendications précédentes, comprenant les stades consistant à préparer une première émulsion en mélangeant le composé organique fortement fluoré avec l'huile en présence de tout ou partie de l'agent tensio-actif et d'une partie de l'eau, et à préparer une émulsion finale en émulsionnant la première émulsion, le reste de l'eau et l'agent tensio-actif éventuellement restant.

14. Procédé selon l'une quelconque des revendications précédentes, comprenant en outre le stade consistant à stériliser l'émulsion récupérée au stade (b) en la chauffant aux environs de 115°C, cette émulsion conservant sa stabilité dans ces conditions.
